# EUROPEAN PATENT APPLICATION

(11) **EP 3 545 896 A1**
(43) Date of publication of application: **02.10.2019**
(21) Application number: 18165293.4
(22) Date of filing: 30.03.2018
(51) Int. Cl.: A61B 34/20, G01S 1/70

(54) **MONITORING OF MOVING OBJECTS IN AN OPERATION ROOM**

(71) Applicant: Koninklijke Philips N.V., 5656 AE Eindhoven (NL)
(72) Inventor: HOLTHUIZEN, Ronaldus Frederik Johannes, 5656 AE Eindhoven (NL); DRIES, Johan Juliana, 5656 AE Eindhoven (NL); HOMAN, Robert Johannes Frederik, 5656 AE Eindhoven (NL); VAN DE GIESSEN, Martijn, 5656 AE Eindhoven (NL); VUURBERG, Edward, 5656 AE Eindhoven (NL); HENDRIKS, Bernardus Hendrikus Wilhelmus, 5656 AE Eindhoven (NL); SPLIETHOFF, Jarich Willem, 5656 AE Eindhoven (NL)
(74) Representative: de Haan, Poul Erik

(57) **Abstract**

A system is suggested comprising a main tracking device and a processing unit. The main tracking device includes a plurality of light sources and a plurality of light detectors. On the basis of light pulses, the main tracking device is configured to determine a current 3D position of a plurality of objects in a room. The processing unit is configured to process image information of each of the objects together with the position information so as to generate a visualization of the image information of the objects in spatial relation to each other.

## Description

### FIELD OF THE INVENTION

The invention generally relates to aspects of assisting a physician during an intervention. Furthermore, the invention relates to a system providing visualizations which may help a physician in performing an intervention. In particular, the invention relates to a tracking system allowing visualizations taking into account movements of objects being present in an operation room.

### BACKGROUND OF THE INVENTION

Increasingly more surgical procedures are performed minimally invasive. Various solutions have been developed the last couple of years to improve the ease of use and the obtainable accuracy for the surgeons. These solutions offer patient and/or device tracking combined with registration of (pre-operative) CT or MRI images.

For example, optical cameras may be integrated in the detector on an X-Ray system or in an operation light. This solution enables an easy-to-use integration of surgical navigation and both 2D and 3D X-ray imaging. A surgical planned path may be made before or during the operation. The user can more easily align a surgical instrument due to a visualization of a virtual path and 3D data being projected on real-time optical images. Surgical instruments with markers can be tracked by device tracking, and markers on the patient are used to compensate for patient motion.

### SUMMARY OF THE INVENTION

It may be seen as an aim to provide a system allowing an integration of position information related to a plurality of different objects in the operation room so as to adapt a visualization to position changes of objects.

This is achieved by the subject matter of each of the respective independent claims. Further embodiments are described in the respective dependent claims.

In general, a system in accordance with an embodiment comprises a main tracking device and a processing unit. The main tracking device includes a plurality of light sources and a plurality of light detectors, wherein each of the light sources is configured to emit light pulses and each of the light detectors is configured to detect the light pulses. On the basis of such light pulses, the main tracking device is configured to determine a current 3D position of a plurality of objects in the operation room.

The processing unit is configured to receive for example from a data base image information of each of the objects tracked by the main tracking device. The processing unit is further configured to receive from the main tracking device 3D position information of the objects. Based on a plurality of 3D positions, also an orientation of an object may be determined. Finally, the processing unit is configured to generate a visualization of the image information of the first object in spatial relation to the image information of the second object. As an example, a first object may be a patient and a second object may be an interventional instrument. As a further example, the image information of an object may be a virtual representation of the object or an image generated by the object.

According to an embodiment, the light sources may be fixedly arranged in the operation room, for example at walls and/or the ceiling of the room, and the light detectors may be respectively arranged at the objects. Alternatively, the light detectors may be fixedly arranged in the operation room and the light sources may be mounted on moving objects in that room. It may also be contemplated that both the light sources and the light detectors are fixedly arranged and that light reflectors are arranged at objects which may change their position in the operation room. It is finally mentioned that also any combination of all these arrangements is possible, in particular, when more than two objects are tracked.

According to an embodiment, the system may comprise one or more imaging device. The imaging device may be adapted to generate real-time images of for example an interior of a patient. The imaging device may be a 2D x-ray imaging device, a 3D computer tomography device, a magnet resonance imaging device, an ultrasound device, a hyperspectral video camera or hyperspectral imaging device. With such an imaging device in the system, the processing unit may not only process previously generated images stored on a data base but may also process real-time generated images. It will be understood, that at least two real-time generated images, or real-time generated and stored images, or more than one stored images may be registered with each other and may thus be displayed in combination.

According to an embodiment, the system includes a display device for displaying a visualization generated by the processing unit of the system. As a first example, the display device may be a monitor, wherein a monitor may be movably arranged in an operation room on a support arm attached for example at to ceiling of the operation room, or may be arranged at a floor standing device. According to another example, the display device may be a transparent or at least semi-transparent display device. A transparent display device provides the possibility that for example a surgeon may look through that display device onto the operation field, but may see information visualized in his or her field of view, which information may help performing an intended intervention. The transparent display device may be integrated in a pair of glasses but may also be a flat monitor which can be positioned between the head and the hands of the surgeon so that the surgeon may look onto and through the transparent monitor so as to see both the information on the monitor as well as the hands with an instrument and the patient beneath the monitor. As a further example, the display device may be a projecting device which may project image information onto a surface of a device or of the patient. Alternatively or additionally, the projecting device may project the information directly onto the retina of an eye.

According to an embodiment, the image information of the patient may include 2D x-ray image data, 3D CT image data, 3D MRI image data, ultrasound image data, and/or video image data. It is noted that the image information of patient may be previously generated and stored, for example during a diagnostic process and/or for planning of an intervention, or may be currently generated and processed by the processing unit in real-time.

According to yet another embodiment, the processing unit of the system may be configured to determine a spatial deviation between the current 3D position of one of the objects and an intended 3D position of that object, and to generate an indication of that deviation, for example on the display device. For example, it may be intended to place an x-ray system in a specific position relative to a patient, but the current position of the x-ray system as detected by the main tracking device differs from that intended position. In such a case, in indication may be generated. Firstly, the indication may simply be an alert indicating that the current position is not the intended position. Further, the indication may include information in which way and how far the current position is away from the intended position. Finally, it may be contemplated that the x-ray system is automatically controlled so as to move from the current position to the intended position. It will be understood that such indication may also be provided for any other object or any plurality of objects in the operation room.

It will be understood that not only elements of the system may be tracked by the main tracking device. In fact, each object, present in an operation room, may be tracked by the main tracking device. For example, each and every interventional device, all parts of imaging devices, one or more display device, even a secondary tracking device, an operation light, and a patient table may be tracked by the main tracking device. Finally, also persons like a patient or a physician/surgeon can be tracked by the main tracking device. In a case in which for example the x-ray system is automatically moved, the main tracking device may ensure that no other object is in the moving space of the x-ray system, i.e. may prevent any collision between the moving x-ray system and any other object.

According to a further embodiment, the system may further comprise a secondary tracking device. The secondary tracking device may be a video tracking device, an electromagnetic tracking device, a radio frequency tracking device, and may include optical shape sensing and/or micro electro-mechanical sensors (MEMS). X-ray devices or video cameras may also be utilized for tracking purposes. Assuming that a tracking device may have a somehow restricted field of view, it may be advantageous to have a secondary tracking device at least for tracking a specific element or object in the operation room.

According to another aspect, a software program product is provided which is configured to run on a processing unit of a system as described above. The software program product may include sets of instructions which cause the processing unit to combine 3D position information with image information of one object, wherein the processing unit may receive from the main tracking device the 3D position information of the object and from a data base or a live imaging device the image information representing the object. The same counts for any further object. The software program product may include sets of instructions which cause the processing unit to receive from the main tracking device 3D position information of a second object or of any further object, and to receive image information representing that object. Finally, the software program product causes the processing unit to generate a visualization of the image information of the first object in spatial relation to the image information of the second or further object, wherein the image information of each of the objects is shown at its current 3D position.

According to an embodiment, the software product further includes sets of instructions which cause the processing unit to select objects to be tracked by the main tracking device, and to select image information of each of the objects from available image information. On the one hand, each moving object may automatically be tracked by the main tracking device so as to avoid any collisions. In other words, the software program product may include sets of instructions which cause the processing unit to generate an alert output in case of the possibility of an impermissible intersection of the tracked objects.

On the other hand, a user like a surgeon may provide an input to the processing unit identifying the object to be tracked. It will be understood that such an input may be provided in any known way, for example via a keyboard, touchpad, computer mouse, touch screen, voice command, or gesture.

In an embodiment including any imaging device, the software program product may further include sets of instructions which cause the processing unit to receive live image information of an object, and to generate a visualization including the live image information.

A corresponding computer program product may preferably be loaded into a work memory of the processing unit. The processing unit may thus be equipped to carry out at least a part of the procedural steps described herein. Further, the invention relates to a computer-readable medium such as a CD-ROM at which the computer program product may be stored. However, the computer program product may also be presented over a network like the World Wide Web and can be downloaded into the working memory of the processing unit from such a network.

According to yet another aspect, a method of using the above-described system is provided. The method may include the steps of determining a 3D position of a first object by means of a main tracking system based on emitting and detecting light pulses, determining a 3D position of a further object by means of the main tracking system based on emitting and detecting light pulses, receiving image information of the first object and of the second/further object, and generating a visualization including the image information of the first object in spatial relation to the image information of the second object.

According to a further embodiment, the method does not include any step of treatment of a human or animal body by surgery. For example, the method does not include a step of inserting an interventional device into a patient. Although the visualization may be generated in real time and in parallel to a surgical procedure, the method does not comprise a step of any incision into tissue and also not any step of resection of tissue, for example of tumour tissue.

It has to be noted that embodiments are described with reference to different subject-matters. In particular, some embodiments are described with reference to method type claims (computer program) whereas other embodiments are described with reference to apparatus type claims (system). However, a person skilled in the art will gather from the above and the following description that unless other notified in addition to any combination of features belonging to one type of subject-matter also any combination between features relating to different subject-matters is considered to be disclosed with this application.

The aspects defined above and further aspects, features and advantages of the present invention may also be derived from the examples of embodiments to be described hereinafter and are explained with reference to examples of embodiments. The invention will be described in more detail hereinafter with reference to examples of embodiments but to which the invention is not limited.

### BRIEF DESCRIPTION OF THE DRAWINGS

Fig 1 illustrates a system in an operation room according to an embodiment.
Fig. 2 shows a light source of a main tracking device according to an embodiment.
Fig. 3 shows a light detector of a main tracking device according to an embodiment.
Fig. 4 is a flow chart illustrating steps of a method according to an embodiment.

The illustration in the drawings is schematically only and not to scale. It is noted that similar elements are provided with the same reference signs in different figures, if appropriate.

### DETAILED DESCRIPTION OF EMBODIMENTS

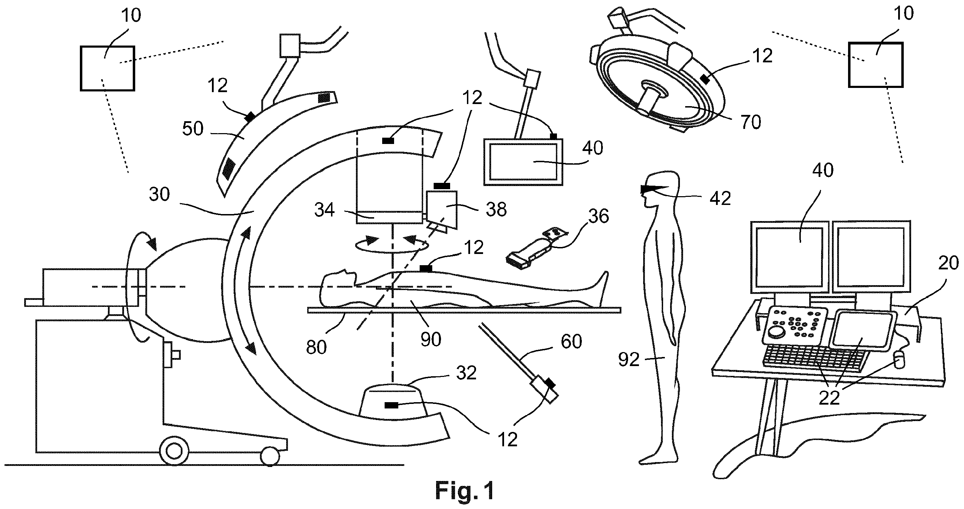

Figure 1 shows an embodiment of a system which may be used in an operation room. The system according to this embodiment includes a main tracking system with two light sources 10 and several light detectors 12. As can be seen in figure 1, the light detectors 12 may be arranged at more or less all movable elements in the operation room. For example, the light detectors 12 may be arranged at an operation light 70, a C arm-based x-ray device 30 or at a monitor 40 which may be attached on a movable support arm. The system of figure 1 further includes a video camera 38, an ultrasound device with a handpiece 36, a secondary tracking device 50, as well as a processing unit 20 with input devices 22. It is noted that the system may include further elements like movable robotic arms (not shown) which may also be tracked. A physician 92 may act in that operation room and must be aware of all parts surrounding him or her. The patient 90 is supported by a patient table 80.

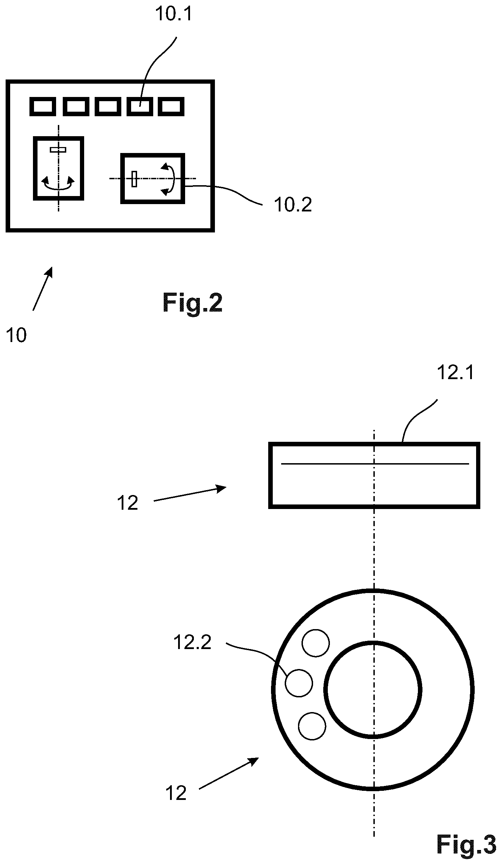

As can be seen in figure 1, even on the patient 90, a detector 12 of the main tracking system may be placed. In figures 2 and 3, examples of a light source 10 and a light detector 12 are shown. The light source 10 according to the embodiment of figure 2 comprises one or more flash lights 10.1 as well as rotatable elements 10.2. The light detector according to figure 3 may include a photo detector 12.1 as well as elements 12.2 which may be trackable by a secondary tracking device.

When operating, the main tracking system may be arranged inside the operation room and may fill the room with light having a wavelength outside the visible light, for example, infra-red light. From these signals, a spatial position of any object in the room can be identify by triangulation. The idea is that instead of measuring angles, the main tracking device emits light beams from elements 10.2, for example from spinning mirrors, inside the light source 10. The rotation rate must be kept very constant and thus time differences can be measured. A light flash may be used for synchronization, wherein that light flash may be emitted from light emitters 10.1, which may be LED flashlights. After the flash for synchronization, two beams sweep in the X and Y direction across the room, which beams may be emitted from elements 10.2 of the light source 10. Based on the time difference between the flash and the sweeps, a microcontroller may calculate the position of each of the light detectors, i.e. of for example photo detectors 12.1 of the light detector 12. No network communication, no centralized server, or anything else is needed. The simple passive detection of the sweeps does most of the work.

The main tracking system may detect an object within 5 cm, preferably within 1 cm and most preferred within 1 mm at a range of 5 m, i.e. most preferred within an angle of 200 microradians. In other words, an object can be detected within an angle subtended by the width of a piece of typing paper held out at arm length. When using light in a specific range of wavelengths, it may also be possible to detect the light beneath a skin of a patient. A light spectrum outside the visible spectrum or at least partly within the red light spectrum would be suitable. That is, the light detector or the light emitter may be implantable or may be at a portion of an instrument which is configured to be temporarily inserted into a patient's body, at least a few millimeters beneath the outer skin.

A spinning cylinder with mirrors emitting a laser sweep may rotate with a frequency of 60 Hz. The measuring time of one cycle may thus be within 48 MHz, that is within usual frequencies of a microcontroller. It should be ensured that the rotating mirror (e.g. element 10.2) spins at a constant rate. The more constant that rate of spinning, the higher the precision of measurement.

Back to figure 1, there is shown the C arm-based x-ray device 30 with an x-ray source 32 and an x-ray detector 34, which may rotate in several directions as indicated by the arrows. Furthermore, the x-ray device may be adapted to move on the floor of the operation room along any path, for example from a rest position to the patient table or along the patient table. In this embodiment, a video camera 38 is attached at the x-ray detector 34, wherein that video camera may be used to observe the surface of the patient 90. The video camera signal may be used to image the operation field and/or as a secondary tracking device. For example, the image of the video camera may be used to identify an orientation of an object, wherein a 3D position of that object is determined by the main tracking device. Video images may be generated parallel to x-ray images generated by the x-ray device. An interventional instrument 60 may also be provided with a light detector 12 so that the main tracking system may detect the three-dimensional position of the instrument.

In the embodiment of the system in figure 1, the secondary tracking device 50 and the operation light 70 are respectively attached, for example, to the ceiling of the operation room by way of movable supporting arms. Besides the main and the secondary tracking device, information about the position and/or the orientation of elements may be received from sensors, for example micro electromechanical sensors (MEMS) which allow for detection of a relative position of elements of one device with respect to each other or which might detect accelerations or movements. For example, the x-ray device 30 may have sensors which allow a determination of the current position of the C arm relative to the base of the x-ray device, and in consequence (knowing the dimensions of the device) of the x-ray source 32 and the x-ray detector 34. On the other hand, the patient table 80 may also be equipped with sensors identifying the height and possibly also a position of the supporting plate relative to the stand of the table, in a case in which the supporting table may be movable for example in a longitudinal direction of the patient 90 relative to other systems in the room.

A first aspect of the main tracking device is to ensure that the movable elements in the operation room do not accidentally collide. That is, the main tracking device may detect a movement of for example the operation light 70 relative to the C-arm of the x-ray device 30, and a rotation of the C-arm may result in a collision of the C-arm with the operation light. In such a case, the processing unit 20 which processes all information from all elements in the room, may generate an alert and may even automatically stop the movement of the C-arm of the x-ray device to avoid any damaging of elements. Tracking the patient or the physician allows to prevent any accident.

Another aspect of the system can be seen in a real-time combination of different images from different image sources. For example, the video camera 38 or the ultrasound device 36 as well as the x-ray system 30 may provide live images and further images may be received from a data base (not shown). A combination of such images, for example as an overlay of images, requires a registration of the images relative to each other. In one embodiment, an element which allows for such a registration and combination of, for example, x-ray images with video images maybe the light detector 12 of the main tracking system which may be positioned on the patient 90. As shown in figure 3, the light detector 12 may include a photo detector 12.1 as an element of the main tracking device, which allows for a detection of the position of the patient in the room. The detection of the position is accurate enough so that even a breathing movement of the chest of the patient may be detected with such a detector. The specific shape of the detector 12 in this embodiment allows, at the same time, a recognition of this specific shape in an x-ray image. Preferable, the detector is formed from a radiopaque material which is visible in an x-ray image. The detector 12 at the patient 90 may also be identified in a video camera image. A segmentation algorithm may be used to calculate the position of the photo-detector, or an imaging marker with a known spatial relationship to the photo-detector. If sufficient photo-detectors are identified in the imaging data, the relationship of the imaging data to the photo-detectors can be calculated. The processing unit 20 may combine a surface image of the patient 90 with x-ray information from within the patient and all this with a synchronized viewing angle and with the video image in the same scale as the x-ray image. Such combined image can be displayed on the monitor 40, either on a stationary monitor at a console or on a movable monitor 40 as shown in figure 1.

A visualization of combined images may also be displayed on a special pair of glasses 42 which are wearable by a physician 92. It will be understood that such glasses are at least semi-transparent so that the physician may look through the glasses onto the patient and onto the operation field. At the same time, those transparent glasses may show information like internal structures of the patient which has been imaged by the x-ray device. It will be understood that such information may also be previously generated and received from a storage like a data base. When using such glasses, which may be called 'augmented reality glasses', the current position of the glasses relative to the patient table or the patient may be of interest. The glasses 42 may be tracked by the main tracking system and thus a spatial position of the glasses in relation to the position of other objects in the room, particularly the patient, can be detected and may thus be known. This allows to identify a viewing direction of the physician in the operation room and thus allows for an adaptation of the C-arm to the viewing angle of the physician and thus for corresponding visualizations of internal structures of the patient viewed, from the same direction.

In the following, some specific embodiments will be discussed which may illustrate examples realized by selected elements of the system of figure 1.

According to an embodiment, the imaging device 30 and the patient 90 may be tracked by the main tracking device, and the imaging of the imaging device is improved by use of the tracking information. For example, a mobile x-ray system may move through the room and makes multiple shots of the legs of the patient in different positions. These images may then be stitched together by using the tracking information. Similarly, a 3D reconstruction based on a plurality of x-ray images from different imaging directions may be brought together.

The tracking of a mobile C-arm may also allow for an overlay of pre-op data or annotations on live fluoroscopy data, for a pre-op data overlay, for a pre-op planning overlay, and also for guidance in orthopedy. It may also allow for park & comeback function (using motorized wheels / base).

The tracking of a fixed C-arm, i.e. not mobile base of a C-arm may allow for calibration free 3D roadmapping and reconstruction, potentially including MEMS sensors and smart modelling, a light-weight less rigid C-Arm or even a system without a C-arm (separate source and detector with individual arms) that can perform roadmapping and 3D reconstruction, improved stroke imaging, and for automatic positioning of the C-Arm or auto-iso-centering.

The tracking of assets like microscope, C-arm, mobile CT system, echo probes, hyperspectral cameras, etc., in the operation room allows for collision avoidance.

By placing photo-detectors or light emitting boxes on known locations on the medical imaging device, the spatial relationship between the patient detectors and the imaging device can be established. This enables determining relationships of imaging data to the photo detectors on the patient without the need of having the markers visible in the actual imaging data itself (e.g. for X-ray fluoroscopy or ultrasound imaging).

According to an embodiment, an interventional instrument may be tracked in addition to the patient. An element of the main tracking device may be placed on the instrument. The angle between the instrument and the tracking markers at the patient may thus be calculated. It is noted that the position of the tracking marker at the patient may also be determined by a secondary tracking device.

According to another embodiment, a wearable display device may be tracked by the main tracking device, wherein a determination of an angular orientation of the wearable display may be of interest. The placement of a light emitting box on the headset can increase the angular accuracy of the tracking of the display with respect to the patient. The camera inside the headset may also be used to detect (passive) optical markers on the patient. The position of the tracked wearable display may further be used to control a navigation system. Examples are the layout of the screen of the surgical navigation software or automated positioning of the detector to avoid collisions and enable better working space.

In a further embodiment, the light emitting device may be configured so as to be implantable. This may facilitate a position localization of the marker. It is noted that significant miniaturization of the light emitting box is possible using MEMS technology and LED's. To obtain a miniaturized broadband light source, a solid-state based light source (such as LED) can be used in combination with inorganic phosphors and optical ceramics. It is also noted that spectral lighting can be used to obtain information about the tissue between the light source and detectors. When using many detectors, it may be possible to calculate 3D spectral reconstruction of the tissue in real-time. Also with a single sensor or a limited set of sensors, 3D spectral data can be acquired by utilizing a more complex light emitting pattern (e.g. instead of a flash followed by 2 laser sweeps, use a flash followed by more distinctive optical patterns such as a scanning laser pattern and/or a larger number of laser sweeps in different directions).

Since all light sources are dimmed w.r.t. patient, the sensitivity of the photo-detector can be significantly increased, compensating for the limited penetration-depth of light in tissues. Inside the implantable marker the measured timings (or the calculated position) are communicated to the outside of the patient using either a wire (that also provides power to the implant) or RF-based communication (Bluetooth etc.) when using a battery powered implantable marker. The implantable marker can also be powered by wireless power transfer based on for instance electromagnetic coupling in the near or midfield. Alternatively, the light detector may be configured so as to be implantable.

It will be understood that it is possible to combine and fuse the information of the tracking information with other sensor data such as inertial (e.g. accelerometers, gyroscopes) sensors. This enables increased accuracy and update speeds of the localization.

According to another embodiment, calibration-free 3D scanning and patient motion compensation is realized.

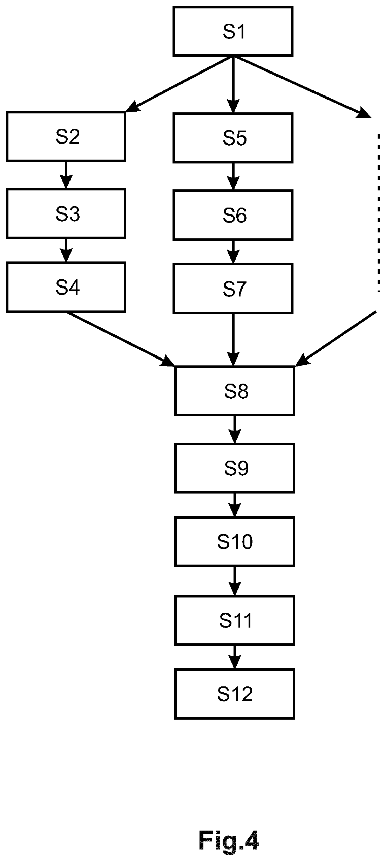

The flow-chart in Fig. 4 illustrates the principle of the steps performed in accordance with an embodiment described herein. It will be understood that the steps described, are major steps, wherein these major steps might be differentiated or divided into several sub-steps. Furthermore, there might be also sub-steps between these major steps.

Assuming that the operation room is already equipped with a main tracking device, for example with light emitters at the walls and light detectors at all relevant objects, the following steps may be performed, with reference to figure 4.

In step S1, the objects which should be tracked are selected, either by a concrete manual selection or automatically by a general indication of the intended procedure.

In step S2, the 3D position of a first object is determined. The 3D position information may also include 3D orientation information. The step may be performed utilizing at least the main tracking device.

In step S3, image information of the first object are received, either from a data base or as live image from an imaging device.

In step S4, the position information of step S2 is tagged to the image information of step S3.

Steps S5 to S7 correspond to steps S2 to S4, but are performed with respect to a second object. It will be understood that such steps may further be performed with respect to further objects.

In step S8, the image and position information of both objects are processed so as to generate a combination of the image information in correct spatial position relative to each other. The combination of image information may be an overlay image of different kinds of images, like a 2D projection from a 3D x-ray image reconstruction onto a video image of an outer surface of a patient, both in the same viewing direction.

In step S9, which is an optional step, an alert is generate, should the processing unit determine a risk of a collision of objects, based on the tracking information.

In step S10, which is also an optional step, at least a stop of movement or an active movement of at least one of the objects may be initiated for collision prevention.

In step S11, a visualization of the combined image information is generated.

In step S12, the visualization is displayed on a display device like a monitor or wearable glasses.

It is noted that most of the steps may be implemented as steps of a computer software, and may thus be performed automatically. It is further noted that the processing unit for processing those steps may be a single processing unit, but may also include a plurality of processing units with separated tasks. For example, the main tracking device may have a first processing unit for determining the 3D positions of all tracked objects. Alternatively or additionally, the visualization may be generated by a separate display processing unit.

While the invention has been illustrated and described in detail in the drawings and foregoing description, such illustration and description are to be considered illustrative or exemplary and not restrictive; the invention is not limited to the disclosed embodiments. Other variations to the disclosed embodiments may be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 10: light source of main tracking device
- 10.1: LED flash light
- 10.2: Laser sweep element
- 12: light detector of main tracking device
- 12.1: photo detector
- 12.2: elements trackable by a secondary tracking device
- 20: processing unit
- 22: input device
- 30: x-ray imaging device
- 32: x-ray source
- 34: x-ray detector
- 36: ultrasound probe
- 38: video camera
- 40: display device
- 42: augmented reality glasses
- 50: secondary tracking device
- 60: interventional instrument
- 70: operation light
- 80: patient table
- 90: patient
- 92: physician

## Claims

1. A system for use in an operation room, the system comprising:
a main tracking device including a plurality of light sources (10) and a plurality of light detectors (12), wherein each of the light sources is configured to emit light pulses and each of the light detectors is configured to detect the light pulses, wherein the main tracking device is configured to determine a current 3D position of a plurality of objects in the operation room on the basis of said light pulses, and
a processing unit (20), wherein the processing unit is configured to receive image information of at least two objects, to receive from the main tracking device 3D position information of the plurality of objects, and to generate a visualization of the image information of a first object in spatial relation to the image information of a second object.

2. The system of claim 1, wherein the light sources (10) are fixedly arranged in the operation room and the light detectors (12) are respectively arranged at the objects.

3. The system of any one of claims 1 and 2, wherein the system further comprises an imaging device (30), wherein the imaging device is at least one device from the group consisting of a 2D x-ray imaging device, a 3D computer tomography device, a magnet resonance imaging device, an ultrasound device (36), a hyperspectral imaging device, and a video camera (38).

4. The system of any one of claims 1 to 3, wherein the system further comprises a display device (40) configured to display the generated visualization.

5. The system of claim 4, wherein the display device is a transparent display device (42).

6. The system of any one of claims 1 to 5, wherein the system further comprises a secondary tracking device (50), wherein the secondary tracking device includes at least one out of the group consisting of a hyperspectral tracking device, a normal video tracking device, an electromagnetic tracking device, a radiofrequency tracking device, an ultrasound tracking device, an x-ray tracking device, an optical shape sensing device, and micro electro-mechanical sensors.

7. The system of any one of claims 1 to 6, wherein the plurality of objects includes at least two out of the group consisting of at least one interventional device (60), the imaging device (30), an imaging registration device, the display device (40, 42), the secondary tracking device (50), an operation light (70), a patient table (80), and at least one person like a patient (90) or a physician (92).

8. The system of any one of claims 1 to 7, wherein the image information of the patient includes at least one out of the group consisting of 2D x-ray image data, 3D CT image data, 3D MRI image data, ultrasound image data, video image data.

9. The system of any one of claims 1 to 8, wherein the processing unit is further configured to determine a spatial deviation between the current 3D position of one of the objects and an intended 3D position of that object, and to generate an indication of that deviation on the display device.

10. A software program product configured to run on the processing unit of a system of claim 1, the software program product including sets of instructions which cause the processing unit
to receive from a main tracking device 3D position information of a first object,
to receive image information representing the first object,
to receive from the main tracking device 3D position information of a second object,
to receive image information representing the second object,
to generate a visualization of the image information of the first object in spatial relation to the image information of the second object.

11. The software product of claim 10, further including sets of instructions which cause the processing unit to select objects to be tracked by the main tracking device, and to select image information of each of the objects from available image information.

12. The software product of claim 10 or 11, further including sets of instructions which cause the processing unit to receive live image information of an object, and to generate a visualization of the first object and the second object including the live image information.

13. The software product of any one of claims 10 to 12, further including sets of instructions which cause the processing unit to generate an alert output in case of an impermissible intersection of the tracked objects.

14. A method of using the system of claim 1, the method including the steps of
determining a 3D position of the first object by means of a main tracking system based on emitting and detecting light pulses,
determining a 3D position of a second object by means of the main tracking system based on emitting and detecting light pulses,
receiving image information of the first object and of the second object,
generating a visualization including the image information of the first object in spatial relation to the image information of the second object.
